# EUROPÄISCHE PATENTANMELDUNG

(11) **EP 1 025 861 A1**
(43) Veröffentlichungstag der Anmeldung: **09.08.2000**
(21) Anmeldenummer: 99101643.7
(22) Anmeldetag: 04.02.1999
(51) Int. Cl.: A61K 47/48, A61K 9/16, A61K 38/17

(54) **Pharmazeutische Zusammensetzung von hydrophob modifizierten Hedgehog-Proteinen und deren Verwendung**

(71) Anmelder: Roche Diagnostics GmbH, 68298 Mannheim (DE)
(72) Erfinder: Die Erfindernennung liegt noch nicht vor

(57) **Zusammenfassung**

Aufgabe der Erfindung ist es, eine pharmazeutische Zusammensetzung eines hydrophob modifizierten Hedgehogproteins mit einem biokompatiblen Träger zur Verfügung zu stellen, wobei der Träger das Hedgehogprotein in der aktiven, gefalteten Struktur bindet und lokal in vivo in aktiver Form verzögert freisetzen kann. Derartige Formulierungen sind insbesondere für die Reparatur von Knochen- und Knorpeldefekten geeignet, können aber auch für die Reparatur neuronaler Defekte oder für ein systemisches Delivery verwendet werden.

Die Aufgabe wird gelöst durch eine pharmazeutische Zusammensetzung eines hydrophob modifizierten Hedgehog-Proteins, welche dadurch gekennzeichnet ist, daß diese hydrophob modifizierte Hedgehog-Proteine und ein biodegradierbares Protein oder ein proteolytisches Abbauprodukt davon als Träger enthält.

## Beschreibung

Gegenstand der Erfindung ist eine pharmazeutische Zusammensetzung von hydrophob modifizierten Hedehog-Proteinen und deren Verwendung, insbesondere zur lokalen Freisetzung dieser Proteine am Knochen.

Unter Hedgehog (hh)-Proteinen versteht man eine Familie von sekretierten Signalproteinen, die verantwortlich sind für die Ausbildung einer Vielzahl von Strukturen in der Embryogenese (J.C. Smith, Cell 76 (1994) 193- 196, N. Perrimon, Cell 80 (1995) 517 - 520, C. Chiang et al., Nature 83 (1996) 407, M.J. Bitgood et al., Curr. Biol. 6 (1996) 296, A. Vortkamp et al., Science 273 (1996) 613, C.J. Lai et al., Development 121 (1995) 2349). Bei der Biosynthese wird nach Abspaltung der Signalsequenz und autokatalytischer Spaltung eine 20 kD N-terminale Domäne und eine 25 kD C-terminale Domäne erhalten. Die N-terminale Domäne ist in ihrer natürlichen Form Cholesterol-modifiziert (J.A. Porter et al., Science 274 (1996) 255 - 259). In höheren Lebewesen besteht die hh-Familie zumindest aus drei Mitgliedern, nämlich Sonic, Indian und Desert hh (Shh, Ihh, Dhh; M. Fietz et al., Development (Suppl.) (1994) 43 - 51). Für Hedgehog-Proteine, welche rekombinant hergestellt wurden, wurde eine unterschiedliche Aktivität nach Herstellung in Prokaryonten und Eukaryonten beobachtet (M. Hynes et al., Neuron 15 (1995) 35 - 44 und T. Nakamura et al., Biochem. Biophys. Res. Comm. 237 (1997) 465 - 469.

Hynes et al. vergleichen die Aktivität von hh im Überstand von transformierten "human embryonic kidney 293 cells" (eukaryontischer hh) mit aus E.coli hergestellten hh und finden eine vierfach höhere Aktivität des hh aus den Überständen der Nierenzellinie. Als Ursache dieser erhöhten Aktivität wird ein potentieller zusätzlicher "accessory factor", der nur in eukaryontischen Zellen exprimiert wird, eine posttranslationale Modifikation, ein unterschiedlicher N-Terminus, der aus E.coli isolierte hh enthält 50 % eines hhs, welcher zwei zusätzliche N-terminale Aminosäuren (Gly-Ser) trägt, oder um 5 - 6 Aminosäuren verkürzt ist, oder in einem höheren Aggregatzustand (z. B. durch Bindung an Nickel-Agarose beads) diskutiert.

Nakamura et al. vergleichen die Aktivität von shh im Überstand von transformierten "chicken embryo fibroblasts" mit einem aus E.coli isolierten shh Fusionsprotein, das noch einen N-terminalen Polyhistidinteil aufweist. Das shh im Überstand der Fibroblasten hat bezüglich der Stimulation von alkalischer Phosphatase (AP) in C3H10T ½ Zellen eine 7-fach höhere Aktivität als das gereinigte E.coli Protein. Als Ursache der erhöhten Aktivität werden Moleküle, wie beispielsweise bone morphogenetic Proteins (BMPs) diskutiert, die nur im Überstand von eukaryontischen Zellen vorhanden sind und die stärkere Induktion der AP verursachen.

Von Kinto et al., FEBS Letters, 404 (1997) 319 - 323 wurde beschrieben, daß hh sekretierende Fibroblasten bei i.m. Implantation auf Kollagen eine ektopische Knochenbildung induzieren. Hedgehog-Proteine besitzen also eine osteoinduktive Aktivität.

Aus der WO 90/08551 ist ein Verfahren zur Herstellung von Deliverysystemen für Proteine mit verzögerter Freisetzung, unter Verwendung von Alginat, bekannt. Dabei wird ein Zweiphasensystem ausgebildet, wobei die erste Phase eine hohe Konzentration des Proteins (gesättigte Lösung) und die zweite Phase Alginat enthält. Eine solche Phasentrennung ist jedoch bei der Herstellung von pharmazeutischen Zusammensetzungen in großen Mengen schwierig und aufwendig durchzuführen.

Aus Kikuchi, A. et al., J. Controlled Release 47 (1997) 21 - 29 ist eine pulsatile Freisetzung von Dextran aus Calcium-Alginat-Komplexen bekannt. Die Kopplung von Hedgehog-Proteinen an solche Komplexe wird jedoch von Kikuchi nicht beschrieben.

Robinson, C.J. et al. beschreiben in Trends in Biotechnology 14 (1996) 451 - 452 die intra-ventrikuläre Implantation von Alginat-Mikrosphären als Methode zur lokalen Applikation von NGF oder NGF-sezernierenden Zellen. Eine Anwendung für Hedgehog-Proteine wird jedoch nicht beschrieben.

Downs, E.C. et al. beschreiben in J. Cell. Physiol. 152 (1992) 422 - 429 die Anwendung von Calcium-Alginatkugeln als Deliverysystem für angiogenese Faktoren. Die Verwendung dieses Verfahrens oder dieses Deliverysystems für Hedgehog-Proteine ist jedoch nicht beschrieben.

Crey, C.J. und J. Dowsett beschreiben in Biotechnol. Bioeng. 31 (1988) 607 - 612 die Verwendung von Calcium/Zink-Alginat-Kugeln als Deliverysystem für Insulin. Eine Verwendung dieses Verfahrens zur Herstellung von Deliverysystemen für Hedgehog-Proteine ist jedoch hieraus nicht bekannt.

Aus Yang et al., Development 124 (1997) 4393-4404, ist bekannt, daß für eine pharmazeutisch wirksame in vivo Aktivität am Wirkort im Körper hohe lokale Hedgehog-Konzentrationen über einen Zeitraum von mindestens 16 h vorherrschen müssen. Das von Yang et al. beschriebene Trägersystem wie das Hedgehog-beladene Chromatographie-Medium Affigel CM, die von Marti et al. in Nature 375 (1995) 322-325 beschriebene Ni-Agarose oder das von Lopez-Martinez et al. in Curr. Biol. 5 (1995) 791-796 verwendete Affigel Blue oder die darin verwendeten Heparin-Agarose-Partikel sind für eine pharmazeutische Anwendung nicht geeignet, da sie immunogen sind und entzündliche Reaktionen hervorrufen können.

Von den Erfindern wurde festgestellt, daß auch der von Kinto et al. für hh-exprimierende Zellen beschriebene biokompatible und bioabbaubare Träger Kollagen für eine optimale pharmazeutische lokale Applikation von Hedgehogproteinen ungeeignet ist. Es wurde gefunden, daß bei einer Beladung von Kollagenträgern mit Hedgehog-Proteinen unter physiologischen Bedingungen (pH ca. 7 und im schwach Sauren (bis pH 4,5)) der größte Teil des applizierten Hedgehog-Proteins innerhalb von Minuten von der Matrix freigesetzt wird. Bei Beladung unter sauren Bedingungen (unter pH 4,5) wird ein großer Teil des applizierten Hedgehog-Proteins denaturiert und irreversibel an den Träger gebunden.

In der EP-A 98 101 893.0 ist eine pharmazeutische Zusammensetzung eines Hedgehog-Proteins beschrieben, welche dadurch gekennzeichnet ist, daß das Hedgehog-Protein an einen hydrophilen Träger, der biokompatibel ist, gebunden ist, wobei der Träger ein Polymer ist, welches das Hedgehog-Protein aufgrund von ionischen Wechselwirkungen als negativ geladener Träger bindet, das Hedgehog-Protein bei der Bindung an den Träger nicht denaturiert, unter neutralen Bedingungen mindestens 0,1 bis 2 negativ geladenen Rest pro Monomer enthält, die Ladung in Form von Säuregruppen enthält, ein durchschnittliches Molekulargewicht von mindestens 50.000 Da aufweist und keine Agarose enthält.

Aufgabe der Erfindung ist es, eine pharmazeutische Zusammensetzung eines hydrophob modifizierten Hedgehogproteins mit einem biokompatiblen Träger zur Verfügung zu stellen, wobei der Träger das Hedgehogprotein in der aktiven, gefalteten Struktur bindet und lokal in vivo in aktiver Form verzögert freisetzen kann. Derartige Formulierungen sind insbesondere für die Reparatur von Knochen- und Knorpeldefekten geeignet, können aber auch für die Reparatur neuronaler Defekte oder für ein systemisches Delivery verwendet werden.

Die Aufgabe wird gelöst durch eine pharmazeutische Zusammensetzung eines hydrophob modifizierten Hedgehog-Proteins, welche dadurch gekennzeichnet ist, daß diese hydrophob modifizierte Hedgehog-Proteine und ein biodegradierbares Protein oder ein proteolytisches Abbauprodukt davon als Träger enthält.

Es hat sich überraschenderweise gezeigt, daß hydrophob modifizierte Hedgehog-Proteine reversibel, aktiv und verzögert von einem solchen Träger in vivo freigesetzt werden, ohne daß immunogene und/oder entzündliche Reaktionen in vivo hervorgerufen werden. Der Träger kann dabei ein fester Träger wie ein Schwamm oder ein injizierbarer Träger wie ein Gel sein. Der bevorzugte bioabbaubare Träger ist Kollagen oder das Hydrolyseprodukt Gelatin. Andere faserartige Trägerproteine wie Fibrin oder Elastin sind ebenfalls geeignet und können als intakte Proteinfasern, als solubilisiertes Protein oder als partiell hydrolysiertes Protein eingesetzt werden.

Unter Kollagen gemäß der Erfindung ist vorzugsweise lösliches Kollagen, unlösliches Kollagen (vorzugsweise quervernetzt), AteloKollagen oder Gelatine zu verstehen. Erfindungsgemäß kann das Kollagen als feste Matrix vorliegen (Lyophilisat oder Präzipitat, quervernetzt oder nicht quervernetzt, als Fasern, Dispersion oder als Gel). Besonders geeignet ist rekombinant hergestelltes Kollagen sowie Kollagen Typ I oder Typ II sowie Mischungen davon. Kollagenfasern können beispielsweise gemäß dem britischen Patent Nr. 1204438 hergestellt werden. Lösliches Kollagen kann beispielsweise durch Verfahren, wie sie in den britischen Patenten Nr. 990276 und 1184502 beschrieben sind, hergestellt werden. Das Produkt, welches nach diesen Verfahren hergestellt wird, kann als Microgel bezeichnet werden und enthält eine Mischung von Kollagen verschiedener Aggregationsformen. Hydrolysiertes Kollagen kann vorzugsweise durch Behandlung von Kollagen mit Trypsin erhalten werden. Dabei entsteht eine polydisperse Mischung von Polypeptiden mit einem Molekulargewicht im Bereich zwischen etwa 5000 und 70000. Vorzugsweise wird vor der Behandlung mit Trypsin das Kollagen zunächst beispielsweise durch Hitzebehandlung denaturiert.

Der bioabbaubare Träger, vorzugsweise Kollagen, wird vorzugsweise als Schwamm eingesetzt, wie beispielsweise der Kollagenschwamm Helistat™ der Firma Integra Life Sciences, USA.

Vorzugsweise wird ein Kollagen als Trägermatrix, besonders bevorzugt als lösliche oder unlösliche Trägermatrix verwendet. Besonders bevorzugt besteht die Trägermatrix aus einer Kombination des bioabbaubaren Trägers und einem anionischen Polysaccharid, wie vorzugsweise Hyaluronsäure (sowie chemisch quervernetzte Formen davon), Chondroitinsulfat, Polyvinylsulfat, Keratansulfat, Dextransulfat, Pektin, Carrageenan und andere Hydrocolloide, sulfatiertes Alginat, Dermatansulfat, Alginat, vorzugsweise Calciumalginat, oder Komplexe aus Protein und Polysacchariden, wie sie beispielsweise in US Patent Nr. 4,614,794 beschrieben sind (Fibracol™, Johnson and Johnson, USA), wobei der Gewichtsanteil der geladenen Polysaccharide 10-50% beträgt. Unlösliche Matrix im Sinne der Erfindung bedeutet, daß die Matrix sich in neutraler, gepufferter Lösung in vitro innerhalb von 10 - 20 Stunden bei Raumtemperatur nicht wesentlich zersetzt oder merkbar auflöst. Dabei ist es bevorzugt, wenn der erfindungsgemäß verwendete Träger weniger als 50 %, vorzugsweise weniger als 20 % und besonders bevorzugt im Wesentlichen keinen Anteil eines neutralen Polysaccharids enthält. Besonders geeignet ist die Kombination von Kollagen mit Hyaluronsäure mit einem Molekulargewicht von mindestens 10⁶ Dalton, insbesondere mit einem Molekulargewicht von 4 x 10⁶ Dalton.

Unter einer verzögerten Freisetzung gemäß der Erfindung wird eine Freisetzung des Hedgehog-Proteins in einer pharmakologisch wirksamen Dosis über einen definierten Zeitraum von mindestens 14 Stunden verstanden. Unter pharmakologischer Wirkung wird eine neurologische Wirkung auf Nervenzellen, eine Chondrogenese und/oder -induktion und vorzugsweise Osteogenese und/oder -induktion verstanden, wie sie in Kinto et al., FEBS Letters, 404 (1997) 319-323 für die Knocheninduktion, für die Wirkung auf Nervenzellen durch Miao et al. in J. Neurosci. 17 (1997) 5891-5899 und für die Knorpelzelleninduktion von Stott et al. in J. Cell Sci. 110 (1997) 2691-2701 beschrieben ist.

Vorzugweise wird als Träger ein enzymatisch abbaubarer Träger verwendet, der durch sezernierte Enzyme (z.B. Proteasen) aus den Zellen, an denen die lokale Applikation in vivo erfolgt, abgebaut wird. Die Halbwertszeit des Trägers soll jedoch mindestens 12 h betragen, kann aber durchaus mehrere Wochen betragen. Falls der Träger aus einem Polysaccharid besteht, wird dieser Träger vorzugsweise durch Glycosidasen und in der Zelle vorhandenen und sezernierten Hydrolasen abgebaut. Eine solche Bioabbaubarkeit des Trägers ist jedoch nicht in jedem Fall erforderlich. Falls die Freisetzung zur Behandlung der Osteoporose oder von neuronalen Erkrankungen erfolgt, ist eine Bioabbaubarkeit nicht notwendig. Vorzugsweise sind solche Träger jedoch unter physiologischen Bedingungen schwer löslich und werden dadurch über längere Zeit (mehrere Wochen bis Monate) vom Körper resorbiert.

Zur Herstellung von Trägermatrices, die mit Hedgehog-Proteinen in einer solchen Weise beschichtet sind, daß sie bei einer Lokalapplikation eine ausreichende pharmazeutische Wirksamkeit zeigen, sind Lösungen der Hedgehog-Proteine in hohen Konzentrationen erforderlich. Es hat sich gezeigt, daß pharmazeutisch anwendbare und mit Hedgehog-Protein beschichtete Träger vorzugsweise eine Konzentration des Hedgehog-Proteins von 1 - 20 mg/ml Träger und mehr enthalten sollten. Besonders vorteilhaft sind Träger, welche Hedgehog-Proteine in einer Konzentration von 10 mg/ml Träger (Lösungsvolumen, Gelvolumen oder Schwammvolumen) oder mehr enthalten. Hedgehog-Proteine sind an sich schwer löslich. Ein weiterer Gegenstand der Erfindung ist ein Verfahren zur Herstellung einer mit Hedgehog-Protein beschichteten Trägermatrix, welches dadurch gekennzeichnet ist, daß die Trägermatrix mit einer Hedgehog-Protein-Lösung in einer Konzentration von 3 mg/ml, welche Arginin oder Argininiumionen enthält, inkubiert wird und die so beschichtete Trägermatrix isoliert wird.

Solche Lösungen sind geeignet, Trägermatrices herzustellen, welche Hedgehog-Proteine in pharmazeutisch wirksamen Konzentrationen enthalten und für die pharmazeutische Anwendung geeignet sind. Ein weiterer Gegenstand der Erfindung ist deshalb eine Kollagenträgermatrix, welche 3 mg hydrophob modifiziertes Hedgehog-Protein oder mehr, vorzugsweise 10 mg oder mehr pro ml Trägermatrix und Arginin oder Argininiumionen (vorzugsweise Argininiumsulfat) enthält. Die Konzentration von Arginin ist vorzugsweise zwischen 10 und 500 mmol/l, vorzugsweise im pH-Bereich zwischen 6 und 8.

Unter Aktivität im Sinne der Erfindung ist die Aktivität an alkalischer Phosphatase (Stimulation der Expression der alkalischen Phosphatase) zu verstehen, die das Polypeptid in Säugerzellen induzieren kann (Aktivität im alkalischen Phosphatasetest). Dabei wird eine Maus-Fibroblasten-Zellinie in einem Medium, welches fötales Kälberserum enthält, kultiviert. Anschließend wird sterilfiltrierte Probe zugesetzt, nach ca. 5 Tagen die Zellen aufgeschlossen und in Zellysat alkalische Phosphatase über die Spaltung eines chromogenen Substrats (pNP, p-Nitrophenol) bestimmt (J. Asahina, Exp. Cell. Res. 222 (1996) 38 - 47 und T. Nakamura (1997)).

Unter einem hydrophob modifizierten (lipophilisierten) Hedgehog-Protein, gemäß der Erfindung, ist ein lipophilisiertes sekretiertes Signalprotein zu verstehen, welches für die Ausbildung einer Vielzahl von Strukturen in der Embryogenese verantwortlich ist. Besonders bevorzugt verwendet wird Sonic, Indian- oder Desert hh (Fietz, M., et al., Development (Suppl.) (1994) 43-51). Bevorzugt wird die prozessierte Form (N-terminale mature Domäne) von sonic hh-Protein (EMBL-Datenbank unter Nr. L38518) beschrieben ist, verwendet. Proteine der Hedgehog-Familie zeigen eine ausgeprägte Homologie in der Aminosäuresequenz, weshalb es ebenso bevorzugt ist, solche Nukleinsäuren zu exprimieren, welche für Hedgehog-Proteine codieren, die zu 80% oder mehr homolog mit der oben genannten Sequenz von Sonic Hedgehog-Protein sind.

Die Lipophilisierung erfolgt vorzugsweise durch chemische Modifikation. Vorzugsweise enthält ein solches Hedgehog-Konjugat (vorzugsweise am C- und/oder N-Terminus) kovalent gebunden ein zusätzliches Polypeptid, bestehend aus 10 - 30 vorzugsweise hydrophoben Aminosäuren und/oder solchen Aminosäuren, welche Transmembranhelices bilden. Besonders bevorzugt enthält das zusätzliche Polypeptid 2 - 12 Lysine und/oder Arginine, jedoch keinen Polyhistidinanteil, der zur Reinigung des Konjugats an einer Ni-Chelatsäule geeignet ist. Ebenfalls bevorzugt ist es, (vorzugsweise am C- und/oder N-Terminus) kovalent 1 - 4 aliphatische, gesättigte oder ungesättigte Kohlenwasserstoffreste mit einer Kettenlänge von 10 - 24 C-Atomen- oder Steroide mit einer lipophilen (hydrophoben) Wirkung zu binden. Weiterhin ist es bevorzugt, hydrophobe Thioverbindungen, wie insbesondere Thiocholesterol, und Thioalkane, -alkene über eine oxidativ gebildete Disulfidbrücke (vorzugsweise an den C- und/oder N-Terminus und dort an das N-terminale Cystein) von hh-Proteinen kovalent zu koppeln.

Durch solche lipophilisierenden Reste wird das Protein hydrophobisiert und damit seine Interaktion mit Lipidmembranen von eukaryontischen Zellen, insbesondere von Säugerzellen, verbessert.

Unter einem lipophilisierten Protein, gemäß der Erfindung, ist demnach ein hydrophobisiertes Protein zu verstehen, welches gegenüber einem unmodifizierten Protein eine erhöhte Oberflächenhydrophobizität zeigt, wodurch seine Affinität für apolare Moleküle oder Amphiphile erhöht wird. Die Zunahme des Lipophilisierungsgrades eines Proteins kann durch den Grad der Integration in eine Lipidschicht gemessen werden, wie beispielsweise beschrieben von Haque, Z. et al., J. Agric. Food Chem. 30 (1982) 481. Verfahren zur hydrophoben (lipophilisierenden) Modifikation von Proteinen sind beispielsweise beschrieben von Haque, Z. et al., J. Agric. Food Chem. 31 (1983) 1225 - 1230; Webb, R.J. et al., Biochemistry 37 (1998) 673 - 679; Hancock, J.F., Cell 63 (1990) 133- 139, A Practical guide to membrane protein purification, Ed. G. v. Jagow, Hermann Schägger (1994) (Kapitel 16, Seiten 535 - 554).

Solche hydrophob modifizierten Hedgehog-Proteine sind beispielsweise in der Europäischen Patentanmeldung Nr. 98116733.1 und 98107911.4 beschrieben.

Das humane Sonic Hedgehog-Precursorprotein besteht aus den Aminosäuren 1 - 462 der in der EMBL-Datenbank unter Nr. L38518 beschriebenen Sequenz. Die Aminosäuren 1 - 23 stellen dabei das Signalpeptid dar, die Aminosäuren 24 - 197 die mature Signaldomäne, die Aminosäuren 32 - 197 die um acht Aminosäuren verkürzte Signaldomäne und die Aminosäuren 198 - 462 die autoprozessierte C-terminale Domäne nach autoproteolytischer Spaltung.

Die erfindungsgemäße pharmazeutische Zusammensetzung enthält eine pharmakologisch effektive Dosis des hh-Proteins und kann lokal appliziert werden. Es ist bevorzugt, die erfindungsgemäßen Proteine in Kombination mit anderen Proteinen der Hedgehog-Familie oder Knochenwachstumsfaktoren wie bone morphogenetic proteins (BMPs) (Wozney et al., Cell. Mol. Biol. of Bone, Bone Morphogenetic Proteins and their Gene Expression (1993) Academic Press Inc., 131-167) oder Parathyroidhormonen (Karablis et al., Genes and Development 8 (1994) 277-289) oder insulin-like growth factors (IGF-I oder II) oder transforming growth factors (TGF-β) zu verwenden.

Die Herstellung der pharmazeutischen Zusammensetzung erfolgt in der Weise, daß das lipophilisierte Hedgehog-Protein mit dem Träger oder dem kollagenhaltigen Träger bei einem pH-Wert von 4,5 oder mehr inkubiert wird. Vorzugsweise erfolgt die Inkubation bei neutralem pH-Wert (pH 6 - 8) und vorzugsweise in gepufferter Lösung. Bei pH 4,5 oder höheren pH-Werten bindet das lipophilisierte Hedgehog-Protein durch hydrophobe Wechselwirkung an den Träger. Wenn die Trägermatrix zusätzlich einen hydrophilen Träger enthält, der Proteine über ionische Wechselwirkungen binden kann (beispielsweise ein anionisches Polysaccharid), dann kann das Hedgehog-Protein auch über ionische Wechselwirkungen mit diesem Teil des Trägers in Wechselwirkung treten. Aus diesem Grund kann das Verhältnis von Kollagen und hydrophilem Trägeranteil in weiten Bereichen variieren. Es ist jedoch bevorzugt, wenn das Verhältnis Kollagenanteil zu hydrophilem Trägeranteil 1,5 : 1 oder mehr beträgt.

Zur Herstellung der pharmazeutischen Zusammensetzung ist es weiter bevorzugt, Hilfsstoffe, wie Zucker (Mannitol, Sucrose, Laktose, Glucose, Saccharose, Trehalose, vorzugsweise 20-100 mg/ml) oder Aminosäuren, wie Glycin oder Arginin, Oxidationsschutzmittel wie Methionin sowie Antioxidantien, wie EDTA, Citrat, Polyethylenglycol (1-10 Gew.%), Detergentien, vorzugsweise nicht-ionische Detergentien (vorzugsweise 0,005-1 Gew.%), wie Polysorbate (Tween® 20 oder Tween® 80) oder Polyoxyethylene, antiinflammatorische Wirkstoffe, lokale Anästhetika, Antibiotika und/oder Stabilisatoren, wie Lipide, Fettsäuren, Glycerin, zuzusetzen.

In einer weiteren bevorzugten Ausführungsform ist eine pharmazeutische Zusammensetzung des erfindungsgemäßen Hedgehog-Proteins mit Suramin bevorzugt und diese vorteilhaft verwendbar.

Die pharmazeutische Zusammensetzung kann weitere pharmazeutische Hilfsstoffe enthalten.

In einer bevorzugten Ausführungsform enthält die pharmazeutische Zusammensetzung das lipophilisierte Hedgehog-Protein in einer Konzentration von 0,1-100 mg/ml.

In einer bevorzugten Ausführungsform enthält die pharmazeutische Zusammensetzung zusätzlich einen pharmazeutisch akzeptablen Puffer, der bioverträglich ist, vorzugsweise im Bereich zwischen pH 4 und pH 10, besonders bevorzugt im Bereich zwischen pH 6 und 8, insbesondere bei einem pH-Wert von ca. pH 7. Der pH-Wert der pharmazeutischen Zusammensetzung sollte zweckmäßig größer pH 4 sein, um eine Denaturierung und die Ablösung des im Hedgehog-Protein komplexierten Zinks zu verhindern. Die Konzentration des Puffers beträgt vorzugsweise 1-500 mmol/l, insbesondere 5 - 150 mmol/l und besonders bevorzugt 10-100 mmol/l. In einer geeigneten Ausführungsform wird als Puffer 20 mmol/l Kaliumphosphatpuffer, pH 7,2 oder 100 mmol/l Argininchlorid, pH 7,2 verwendet.

Die folgenden Beispiele, Publikationen und Abbildungen erläutern die Erfindung, deren Schutzumfang sich aus den Patentansprüchen ergibt, weiter. Die beschriebenen Verfahren sind als Beispiele zu verstehen, die auch noch nach Modifikationen den Gegenstand der Erfindung beschreiben.

### Figurenbeschreibung

- **Figur 1:**: In vitro Freisetzung von hydrophob modifiziertem shh aus einer Kollagenmatrix (Helistat™)
- **Figur 2:**: In vitro Freisetzung von Dipalmityl-shh aus einer Kollagen/Alginat-Matrix (Fibracol™)

### Beispiele

### Beispiel 1:

### In vitro-Freisetzung von hydrophob modifizierten hedgehog-Proteinen von einer Kollagenmatrix (Helistat™):

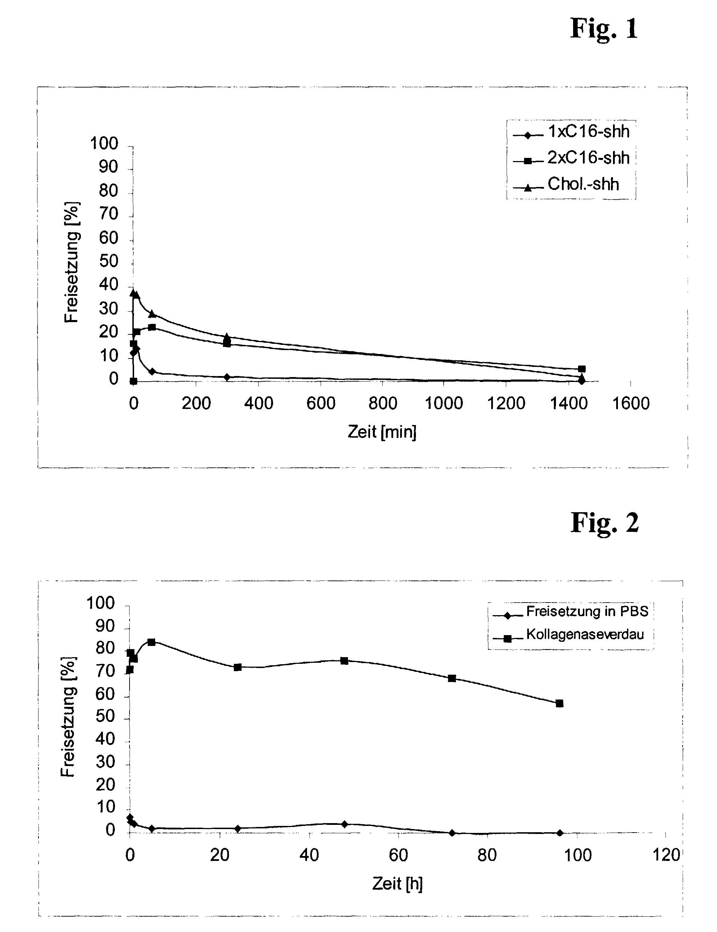

Auf Kollagenschwämmchen (Helistat™, Integra Life Sciences, USA) der Größe 10 x 10 x 3 mm werden jeweils 0.1 ml von Lösungen unterschiedlich modifizierter hedgehog Proteine (Palmityl-shh = 1xC16-shh, Dipalmityl-shh = 2xC16-shh bzw. Cholesteryl-shh = Chol-shh) aufgetropft. Die beladenen Träger werden eingefroren (-70 °C), lyophilisiert und analysiert. Dabei werden die Schwämme in einem geeigneten Volumen PBS bei 37 °C inkubiert. Die Menge an freigesetzem hh wird mittels rp-HPLC bestimmt (siehe Fig.1).

Die Analyse der in vitro-Freisetzungskinetik zeigt, daß Shh aus der Kollagenmatrix über einen Zeitraum von 20 h verzögert oder in geringen Mengen freigesetzt wird (Fig. 1).

Man erkennt, daß in vitro unter den angegebenen Bedingungen nur ca. 20% des geladenen 2xC16-shh freigesetzt werden. Die restlichen 80% des aufgetragenen Proteins können in vivo durch Bioabbau des Trägers freigesetzt werden. Diese Situation kann in vitro durch einen enzymatischen Abbau des Kollagenträgers mittels rekombinanter Kollagenase simuliert werden. Dazu wird ein mit 2xC16-shh beladener Kollagenschwamm der für 14 h unter Freisetzungsbedingungen inkubiert wurde (siehe oben) in 490 µl Puffer (50 mM HEPES-NaOH, 800 mM NaCl, 0.1% Tween, pH 7.4) überführt und mit 10 µl rekombinanter Kollagenase I (1mg/ml, in 10 mM Tris-Cl, 1 mM CaCl₂, pH 7.4) versetzt. Nach einer Inkubation für 2 h bei 37 °C wird das freigesetzte hedgehog-Protein mittels reversed phase-HPLC und in einem in vitro-Aktivitätstest (Induktion alkalischer Phosphatase in C3H10T1/2 Zellen) analysiert. Aus dieser Analyse wird deutlich, daß der unter Freisetzungsbedingungen nicht freigesetzte Anteil von 2xC16-shh (siehe oben) im Kollagenträger verbleibt, mittels Kollagenaseverdau freigesetzt werden kann und seine Aktivität dabei nicht verliert (ca. 80% Wiederfindung).

### Beispiel 2:

### In vitro-Freisetzung von hydrophob modifiziertem hedgehog-Protein (Dipalmityl-shh) von einer Kollagen-Alginat-Matrix (Fibracol™)

Auf Kollagen-Alginat-Schwämmchen (Fibracol™; Johnson & Johnson, USA) der ungefähren Größe 10 x 10 x 3 mm werden jeweils 0.05 ml einer Lösung von Dipalmityl-shh (1 mg/ml) aufgetropft. Die beladenen Träger werden eingefroren (-70 °C), lyophilisiert und analysiert. Dabei werden die Schwämme in einem geeigneten Volumen PBS bei 37 °C inkubiert. Die Menge an freigesetztem 2xC16-shh wird mittels rpHPLC bestimmt (siehe Fig. 2).

Man erkennt, daß in vitro unter den angegebenen Bedingungen nur ca. 10% des geladenen 2xC16 freigesetzt werden. Die restlichen 90% des aufgetragenen Proteins können in vivo durch Bioabbau des Trägers freigesetzt werden. Diese Situation kann in vitro durch einen enzymatischen Teilabbau des Trägers mittels rekombinanter Kollagenase simuliert werden. Dazu werden die mit 2xC16-shh beladenen Träger, die unter Freisetzungsbedingungen inkubiert wurden (siehe oben) in 490 µl Puffer (50 mM HEPES-NaOH, 800 mM NaCl, 0.1% Tween, pH 7.4) überführt und mit 10 µl rekombinanter Kollagenase I (lmg/ml, in 10 mM Tris-Cl, 1 mM CaCl₂, pH 7.4) versetzt. Nach einer Inkubation für 2 h bei 37 °C wird nach einer Zentrifugation das freigesetzte hedgehog-Protein mittels reversed phase-HPLC analysiert. Aus dieser Analyse wird deutlich, daß der unter Freisetzungsbedingungen nicht freigesetzte Anteil von 2xC16-shh im Kollagen-Alginat-Träger verbleibt und mittels Kollgenaseverdau freigesetzt werden kann (ca. 80% Wiederfindung; Fig. 2).

### Referenzliste

A Practical guide to membrane protein purification, Ed. G. v. Jagow, Hermann Schägger (1994) (Kapitel 16, Seiten 535 - 554)
Asahina, J., Exp. Cell. Res. 222 (1996) 38-47
Bitgood, M.J., et al., Curr. Biol. 6 (1996) 296
Britisches Patent Nr. 1184502
Britisches Patent Nr. 1204438
Britisches Patent Nr. 990276
Chiang, C., et al., Nature 83 (1996) 407
Crey, C.J. et al., Biotechnol. Bioeng. 31 (1988) 607 - 612
Downs, E.C. et al., J. Cellular Physiology 152 (1992) 422 - 429
EP-A 98 101 893.0
Europäischen Patentanmeldung Nr. 98107911.4
Europäischen Patentanmeldung Nr. 98116733.1
Fietz, M., et al., Development (Suppl.) (1994) 43-51
Hancock, J.F., Cell 63 (1990) 133 - 139
Haque, Z. et al., J. Agric. Food Chem. 30 (1982) 481
Haque, Z. et al., J. Agric. Food Chem. 31 (1983) 1225- 1230
Hynes, M., et al., Neuron 15 (1995) 35-44
Karablis et al., Genes and Development 8 (1994) 277-289
Kikuchi, A. et al., J. Controlled Release 47 (1997) 21 - 29
Kinto et al., FEBS Letters, 404 (1997) 319-323
Lai, C.J., et al., Development 121 (1995) 2349
Lopez-Martinez et al., Curr. Biol. 5 (1995) 791-796
Marti et al., Nature 375 (1995) 322-325
Miao et al., J. Neurosci. 17 (1997) 5891-5899
Nakamura, T., et al., Biochem. Biophys. Res. Comm. 237 (1997) 465-469
Perrimon, N., Cell 80 (1995) 517-520
Porter, J.A., et al., Science 274 (1996) 255-259
Robinson, C.J. et al., Trends in Biotechnology 14 (1996) 451 - 452
Smith, J.C., Cell 76 (1994) 193-196
Stott et al., J. Cell Sci. 110 (1997) 2691-2701
US Patent Nr. 4,614,794
Vortkamp, A., et al., Science 273 (1996) 613
Webb, R.J. et al., Biochemistry 37 (1998) 673 - 679
WO 90/08551
Wozney et al., Cell. Mol. Biol. of Bone, Bone Morphogenetic Proteins and their Gene Expression (1993) Academic Press inc., 131-167
Yang et al., Development 124 (1997) 4393-4404

## Patentansprüche

1. Pharmazeutische Zusammensetzung, enthaltend ein hydrophob modifiziertes Hedgehog-Protein und ein bioabbaubares Protein als Träger.

2. Pharmazeutische Zusammensetzung nach Anspruch 1, enthaltend lösliches Kollagen als Träger.

3. Pharmazeutische Zusammensetzung nach Anspruch 1, enthaltend unlösliches, quervernetztes Kollagen.

4. Pharmazeutische Zusammensetzung nach den Ansprüchen 1 - 3, enthaltend eine Hyaluronsäure oder Alginat.

5. Pharmazeutische Zusammensetzung nach den Ansprüchen 1 - 4, enthaltend ein Hedgehog-Protein in einer Konzentration von 0,1 - 100 mg/ml.

6. Pharmazeutische Zusammensetzung nach den Ansprüchen 1 - 5, dadurch gekennzeichnet, daß die Zusammensetzung im Bereich zwischen pH 4.5 und 10 gepuffert ist.

7. Pharmazeutische Zusammensetzung nach den Ansprüchen 1 - 6, enthaltend Arginin oder Argininiumionen.

8. Verfahren zur Herstellung einer pharmazeutischen Zusammensetzung, dadurch gekennzeichnet, daß ein hydrophob modifiziertes Hedgehog-Protein in einer therapeutisch wirksamen Menge mit einem bioabbaubaren Protein als Träger kombiniert wird.

9. Verfahren zur verzögerten Freisetzung eines hydrophob modifizierten Hedgehog-Proteins im menschlichen Körper, dadurch gekennzeichnet, daß das genannte Hedgehog-Protein in einer pharmazeutischen Zusammensetzung nach den Ansprüchen 1 - 7 lokal im menschlichen Körper appliziert wird.

10. Verfahren zur Herstellung einer unlöslichen, bioabbaubaren Proteinträgermatrix, welche ein hydrophob modifiziertes Hedgehog-Protein enthält, dadurch gekennzeichnet, daß die Trägermatrix mit einer Lösung, enthaltend das genannte Hedgehog-Protein in einer Konzentration von 3 mg/ml oder mehr und Arginin oder Argininiumionen in einer Konzentration von 10 mmol/l oder mehr inkubiert und die so beschichtete Trägermatrix isoliert wird.
